Europäisches Patentamt

European Patent Office

Office européen des brevets

(19)

(11) Publication number: **0 396 801**

**A1**

(12) **EUROPEAN PATENT APPLICATION**

(21) Application number: **89108493.1**

(22) Date of filing: **11.05.89**

(51) Int. Cl.⁵: **G01N 33/538, G01N 33/58, G01N 33/546, G01N 33/74, G01N 33/68**

(43) Date of publication of application:
**14.11.90 Bulletin 90/46**

(84) Designated Contracting States:
**AT BE CH DE ES FR GB GR IT LI LU NL SE**

(71) Applicant: **TEIKOKU HORMONE MFG. CO., LTD.**
**5-1, 2-chome, Akasaka**
**Minato-ku, Tokyo(JP)**

(72) Inventor: **Manita, Hideaki**
**1-11-20, Misono**
**Sagamihara-shi Kanagawa-ken(JP)**
Inventor: **Takegawa, Toshiko** No. 1-2, **TEIKOKU HORMONE MFG.**
**Shataku, 1198, Kamiodanaka Nakahara-ku**
**Kawasaki-shi Kanagawa-ken(JP)**
Inventor: **Nonaka, Kazuhiko**
**6-12-19, Tamadaira**
**Hino-shi Tokyo(JP)**
Inventor: **Sasamoto, Hidehiko**
**3-26-8, Eifuku Suginami-ku**
**Tokyo(JP)**

(74) Representative: **Hansen, Bernd, Dr.rer.nat. et al**
**Hoffmann, Eitle & Partner Patentanwälte**
**Arabellastrasse 4 Postfach 81 04 20**
**D-8000 München 81(DE)**

(54) **Simple method for immunological assay.**

(57) Using an antibody-carrying membrane having carried a first antibody on a porous carrier membrane and an antibody-carrying latex having carried a second antibody having an antigenic determinant different from the first antibody on latex colored with a color different from that of the carrier membrane, an analyte can be assayed in a simple manner with high sensitivity. This assay method utilizing immunological reaction is characterized by visual determination and judgment of the results by change or contrast in color.

EP 0 396 801 A1

## SIMPLE METHOD FOR IMMUNOLOGICAL ASSAY

### BACKGROUND OF THE INVENTION

### FIELD OF THE INVENTION

The present invention relates to a method for immunological assay which can be visually observed and easily determined, using an antibody-carrying membrane having carried a first antibody on a porous membrane and an antibody-carrying latex having carried a second antibody on latex particles.

### PRIOR ART

In recent years, detection of biosubstances such as hormones, blood factors, chemicals or metabolites thereof or determination of their concentrations in blood, urine or other body fluids are becoming significant for conditions or diagnosis of disease, prognostic judgment, determination of therapy, etc. Assay for these substances includes a physicochemical method, a biochemical method, an immunological method, etc. Among them, the immunological method has been widely applied to measurement of hormones, blood factors and the like since trace components can be specifically assayed with good accuracy. This method generally comprises using an antibody obtained by immunizing animals such as rabbits, guinea pigs, sheep, etc. with the substance to be analyzed (hereafter referred to as "analyte") as an antigen and utilizing its antigen-antibody reaction. Depending upon the means for measurement, procedures, etc., there have been devised and utilized various methods such as gel diffusion, immunoelectrophoresis, precipitation reaction, agglutination, radioimmunoassay, enzymeimmunoassay, etc. Among them, the agglutination reaction or agglutination inhibition reaction utilizing the reaction between sensitized carrier particles having adsorbed an antigen or antibody to fine particles (carrier) such as blood cells, kaolin, bentonite, polystyrene latex, etc. and the corresponding antibody or antigen has been widely applied because the method is simple and has a relatively good sensitivity. Assays using gonadotropic hormones, steroid hormones, fibrinogen, FDP (fibrin-fibrinogen degradation products) and the like have been widely used in the clinical field. As carriers for the simple method for immunological assay, blood cells and polystyrene latex have been used in most cases. In particular, latex agglutination achieves measurement on a glass plate in an extremely short period of time so that the method is simple and especially advantageous for tests requiring emergency. According to the agglutination reaction or agglutination inhibition reaction, however, agglutination of the reacted latex particles should be observed in the presence of the unreacted latex particles. Thus, its reaction pattern becomes often unclear and the judgment is more often difficult. Recently, enzymeimmunoassay (EIA) in which an enzyme is used instead of a radioactive substance in radioimmunoassay has become widely popular. The enzymeimmunoassay is a method which comprises carrying out an antigen-antibody reaction competitively or non-competitively using, e.g., enzyme-labeled antibody, to react the antigen-bound enzyme-labeled antibody or unbound enzyme with substrate, if necessary and desired, further adding a color-forming material, and then colorimetrically determining the amount of the antigen in a sample with a spectrophotometer. In EIA which principally uses a sandwich obtained by immobilizing a first antibody onto a membrane, a tube, etc., a much higher sensitivity can be obtained than in the aforesaid agglutination reaction or agglutination inhibition reaction. However, EIA requires 3 step reactions: a reaction between the first antibody and a sample, a reaction between the second antibody and enzyme-linked substance and a color forming reaction so that the reaction time is prolonged and the operation is complicated. Furthermore, as a method utilizing enzymeimmunoassay through visual determination without using a colorimeter, there is known a method which comprises keeping and immobilizing antibody-sensitized particles within fibrous porous matrix; causing a reaction with an antigen in a liquid sample on the antibody-sensitized fibers; washing and further reacting with an enzyme-labeled antibody; rinsing to remove an excess of the enzyme-labeled antibody; then adding a substrate solution to form a color on the fibers; and detecting the analyte in the liquid sample with the formed color (cf. EPC Patent $A_2$ Publication No. 217403).

In the enzymeimmunoassay, the presence of the analyte in a liquid sample can be recognized by change in color as described above. However, special devices such as a spectrophotometer, etc. are required for the measurement except for a special case. Furthermore, the use of enzyme involves problems of inactivation and stability during storage. On the other hand, there is disclosed a method using a labeled component obtained by binding the objective component or an agent specific to the objective component to colloidal metal particles (cf. European Patent $A_2$ Publication No. 158746). However, the method using colloidal metal particles requires heating for the purpose of accelerating the reaction and also requires longer periods of time for reaction. Furthermore, in order to enhance the sensitivity, it is necessary to make the contrast clear by using physical developers such as silver lactate, silver nitrate, etc. in colloidal metal particles bound to the surface of blotting media. In addition, these colloidal metal particles are detrimental to stability during storage.

## SUMMARY OF THE INVENTION

Therefore, the present inventors have made investigations on an assay method which can be visually observed and easily determined in a simple operation with high sensitivity. As the result, a method has been found which comprises using an antibody-carrying membrane having carried a first antibody on a porous carrier membrane with a pore diameter larger than a particle diameter of latex particles and an antibody-carrying latex having carried a second antibody having an antigenic determinant different from the first antibody on latex colored with a color different from that of the carrier membrane and the present invention has thus been accomplished.

The present invention relates to an immunological assay for an antigen in a liquid sample which comprises:

reacting an antigen contained in the liquid sample with a first antibody which is carried on a porous carrier membrane (hereafter referred to as "porous membrane" or "carrier membrane") having a pore diameter larger than a particle diameter of latex particles; and,

reacting the formed antigen-antibody complex with a second antibody which is carried on latex particles having a color different from that of the porous membrane and has an antigenic determinant different from the first antibody.

The present invention also relates to a kit for an immunological assay for an antigen in a liquid sample comprising:

a first antibody-carrying membrane in which a first antibody is carried on a porous carrier membrane having a pore diameter larger than a particle diameter of latex particles; and,

a second antibody-carrying latex particles in which a second antibody is carried on latex particles having a color different from that of said carrier membrane and has an antigenic determinant different from said first antibody.

The terms "carried" and "carrying" used herein mean "physically and/or chemically carried" and "physically and/or chemically carrying", respectively.

According to the method and the kit of the present invention, a reaction time can be shortened and a high sensitivity can be achieved and hence, the measurement results can be easily judged visually.

## DETAILED DESCRIPTION OF PREFERRED EMBODIMENTS

Examples of the antigen used to produce antibodies include the following substances.

Peptide hormones, for example, pituitary hormones such as growth hormone (GH), adrenocorticotropic hormone (ACTH), melanocyte stimulating hormone (MSH), prolactin, thyroid stimulating hormone (TSH), luteinizing hormone (LH), follicle stimulating hormone (FSH), oxytocin, human menopausal (urinary) gonadotropin (HMG), etc.; calcium metabolism regulating hormones such as calcitonin, parathyroid hormone, etc.; pancreatic hormones such as insulin, proinsulin, pancreatic glucagon, etc.; gastrointestinal hormones such as gastrin, secretin, etc.; hormons acting on blood vessels such as angiotensin, bradykinin, etc.; placental hormones such as human chorionic gonadotropin (hCG), human placental lactogen (hPL), etc.; and substances other than hormones, for example, enzymes such as prostate acidic phosphatase (PAP), alkaline phosphatase, lactate dehydrogenase, transaminase, trypsin, pepsinogen, etc.; tumor specific substances such as α-fetoprotein (AFP), carcinoembryonic antigen (CEA), etc.; serum protein components such as immunoglobulin (IgG), fibrin-fibrinogen decomposition products (FDP), anti-thrombin III (AT III), transferrin, etc.; rheumatoid factor, serotonin, urokinase, ferritin, substance P, etc.

The antibody used in the present invention can be obtained in a conventional manner by administering an antigen to an animal to immunize, collecting antisera from the animal when an antibody titer reaches a definite level or higher, and purifying the antisera, if necessary and desired. In the present invention, monoclonal antibodies may also be used as the antibody having a different antigenic determinant. The monoclonal antibodies can be produced and collected by producing monoclonal antibody-producing cell line in an appropriate selection and combination of known techniques and utilizing the cell line [for example, see Japanese Patent Application KOKAI No. 20149/1985 (the term "KOKAI" refers to unexamined application laid open to public inspection)]. These antibodies also include fractions of Fab, Fab′, F(ab′)₂ obtained by antibody molecule IgG with enzyme.

As the antibody to be carried on the porous membrane and latex particles, an antibody having a different antigenic determinant to the analyte is used. For example, in the case of using a monoclonal antibody as the first antibody carried on the porous membrane, a monoclonal antibody having an antigenic determinant different from the first monoclonal antibody or a polyclonal antibody to the same antigen is used. Furthermore, in the case of using a polyclonal antibody as the first antibody, a monoclonal antibody or a polyclonal antibody can be used as the second antibody.

As the porous membrane used in the present invention, illustratively shown are filter paper, cellulose acetate membrane, nitrocellulose membrane, immunodyne immunoaffinity membrane, etc. It is necessary that these porous membranes have pores on the surface and the pores be penetrated through from the surface to the back surface of the membrane, whereby the unreacted latex particles can pass through the pores. For this reason, it is required that the pore diameter of the porous membrane be larger than the particle diameter of the latex particles. The pore diameter of the porous membrane is generally approximately 0.2 to 10 μm, preferably about 0.45 to about 10 μm. It is preferred that a ratio of the pore diameter of the porous membrane to the diameter of the latex particles be in a range of 3 : 1 to 15 : 1.

The porous membrane should be colored in such a way that the second antibody-carrying latex bound to the antigen is clearly discernible when the latex particles are white. As these colored membranes, commercially available membrane filter, filter paper and the like are used.

The first antibody is carried on the porous membrane in a conventional manner. To carry the antibody onto, for example, cellulose acetate membrane or nitrocellulose membrane, a solution of the antibody is dropped onto or allowed to immerse the dry membrane; after drying, the unadsorbed portion is blocked with an inactive protein, etc. In addition, there are known a method which comprises activating filter paper with cyanogen bromide to couple with a primary amine and then binding the antibody thereto; and a method which comprises activating with cyanogen bromide likewise, reacting with an ethylenediamine compound at low temperatures to prepare -amino derivatives and then adding the antibody (cf. Fundamental Biochemical Experiment, Series 2, published by Maruzen Publishing Co., Ltd.). To carry the antibody onto immunodyne affinity membrane can also be made in a conventional manner.

As the second antibody-carrying latex used in the present invention, an antibody-carrying latex used in conventional agglutination reaction or agglutination inhibition reaction can be exemplified. Examples of the latex particles include polystyrene latex, styrene-butadiene copolymer latex, polyvinyltoluene latex, vinyltoluene-t-butylstyrene copolymer latex, styrene-methacrylate copolymer latex, etc.; and reactive high molecular latexes containing as functional groups, carboxyl groups, primary amino groups or carbamide groups (-CONH₂) and composed of the aforesaid latexes as the main body; and the like.

The particle diameter of the latex particles can be appropriately chosen but it is desired that those having a mean particle diameter in a range of about 0.01 to about 2 μm, especially about 0.05 to about 0.8 μm.

When the porous membrane described above is white, the latex particles can be commercially available colored latex particles, for example, polystyrene latex particles (red, pink, blue, yellow, green, etc.) manufactured by Japan Synthetic Rubber Co., Ltd. Where the porous membrane is colored with colors other than white, the colored latex particles that are clearly distinguishable when bound to the antigen are used. It is preferred to use the latex particles having a color with strong contrast; for example, where the porous membrane is yellow, the color of the latex particles may be red, blue or green, etc.; when the porous membrane is blue, the color of the latex particles may be white, yellow, etc.

To carry the second antibody to the analyte on the latex particles described above, there may be utilized any of known methods, for example, a method for physically adsorbing antibody to the latex particles and a method of chemically binding the antibody to the latex particles. In the present invention, the second antibody-carrying latex particles refer to all the latex particles having carried thereon the second antibody by appropriately selecting and utilizing these optional methods.

In order to carry the antibody on the latex particles by adsorption, it is sufficient to mix a solution of the antibody with a suspension of the latex particles, whereby the second antibody-carrying latex particles can easily be obtained. In many cases, the antibody contains carboxyl groups and primary amino groups

4

therein. In order to chemically bind the antibody to the latex particles, for example, the reactive high molecular latex containing the functional groups described above is bound to the antibody using, for example, the carbodiimide method.

Examples of reagents for immunochemical assay comprising the combination of the first antibody-carrying membrane and the second antibody-carrying latex particles include the following reagents for assay.

(1) Anti-human chorionic gonadotropin (hCG) monoclonal antibody-carrying nitrocellulose membrane and anti-hCG polyclonal antibody-carrying latex particles;

(2) Anti-prostate acidic phosphatase (PAP) polyclonal antibody-carrying membrane filter and anti-PAP monoclonal antibody-carrying latex particles;

(3) Anti-$\alpha$-fetroprotein (AFP) monoclonal antibody-carrying filter paper and anti-AFP monoclonal antibody-carrying latex particles;

(4) Anti-carcinoembryonic antigen (CEA) polyclonal antibody-carrying immunodyne immunoaffinity membrane and anti-CEA monoclonal antibody-carrying latex particles;

(5) Anti-fibrin/fibrinogen degradation product (FDP) monoclonal antibody-carrying nitrocellulose membrane and anti-FDP monoclonal antibody-carrying latex particles;

(6) Anti-human growth hormone (hGH) polyclonal antibody-carrying filter paper and anti-hGH monoclonal antibody-carrying latex particles;

(7) Anti-human lutenizing hormone (hLH) monoclonal antibody-immobilized membrane and anti-hLH monoclonal antibody-carrying latex particles;

(8) Anti-human menopausal gonadotropin (HMG) antibody-immobilized membrane and anti-hFSH-specific monoclonal antibody-carrying latex particles;

(9) Anti-human placental lactogen (hPL) antibody-immobilized membrane and anti-hPL antibody-carrying latex particles; etc.

In the practice of the present invention, the antigen contained in a liquid sample is reacted with the first antibody carried on the carrier membrane.

To react the antigen with the first antibody, it is preferred to dropwise add a sample solution or its dilution onto the first antibody-carrying membrane. By doing so, the antigen in the liquid sample is bound to the first antibody to form the antigen-antibody complex.

If necessary and desired, after the carrier membrane is washed with water, buffer, etc., the second antibody-carrying latex is dropped to cause a reaction ·between the antigen-antibody complex and the second antibody.

Thereafter, the product is washed with water, buffer, etc. to wash the unreacted latex particles off.

As the buffer, for example, phosphate buffer, Tris-hydrochloride buffer, etc. are used. These buffers may also contain bovine serum albumin (BSA), rabbit serum albumin (RSA), etc. It is preferred to use Tris-hydrochloride buffer containing 0.05 to 0.2% BSA.

Where the antigen is present in the liquid sample, the second antibody-carrying latex particles having a color different from that of the carrier membrane are bound on the carrier membrane. The second antibody-carrying latex particles that are not bound to the antigen are washed off through the pores of the membrane and only the second antibody carrying latex particles that are bound to the antigen remain. The color of the carrier membrane changes at the latex particle-bound portion so that the results can be easily judged visually. When the antigen is absent in the liquid sample, the latex particles carrying thereon the second antibody are all flown out through the pores of the carrier membrane and hence, the color of the carrier membrane does not change. In this case, it may make judgment easier to carry on the carrier membrane the first antibody as discernible shapes like symbols such as a spot having a definite diameter, a star, +, etc. Furthermore, the first antibody-carrying membrane may be immersed in a liquid sample but it is preferred to previously keep the first antibody-carrying membrane in a cylindrical container, preferably in such a device that can adsorb or suck the liquid sample from the bottom, when the liquid sample is dropped from the top.

Example 1

(1-a) Preparation of anti-hCG-specific monoclonal antibody

hCG (10000 iu/mg) was subcutaneously administered to Balb/c mouse at the back 3 times in 3 weeks interval, together with complete Freund's adjuvant. Further 3 weeks after, hCG was intraperitoneally administered. Spleen cells were withdrawn 3 days after the final immunization and fused with myeloma cells (NS-1) in a conventional manner. After selection by HAT, cloning was repeated to give a fused cell line secreting hCG-specific antibody. This fused cell line was intraperitoneally administered to Balb/c mouse previously administered with 0.5 ml of puristan intraperitoneally to form ascites tumor. The ascites was collected and subjected to fractionation with ammonium sulfate and then Affigel-Protein A MAPS KIT, manufactured by Bio-rad Inc. to purify IgG. IgG was freeze dried to give white powdery anti-hCG-specific monoclonal antibody.

(1-b) Preparation of anti-hCG polyclonal antibody

hCG, 1·mg, was subcutaneously administered to rabbit at the back together with complete Freund's adjuvant and one month after, hCG was administered twice at 3 weeks interval subcutaneously and at the paw. Whole blood was collected 3 weeks after the final immunization and sera were separated therefrom to give the antisera. The obtained antisera were immobilized at 56°C for 30 minutes. After purification by fractionation with ammonium sulfate, DEAE-cellulose chromatography and gel filtration by Sephadex G200, the antisera were freeze dried to give white powdery anti-hCG polyclonal antibody.

(1-c) Preparation of anti-hCG-specific monoclonal antibody-immobilized membrane

White nitrocellulose membrane (manufactured by Toyo Filter Paper Co., Ltd., diameter of 20 mm) having a pore diameter of 5 $\mu$m was washed with purified water and then drained. After allowing to stand for 15 minutes in a thermostat at 37°C under humidity of 80%, 2 $\mu$l each of 500 $\mu$g/ml solution (0.9% NaCl-containing 20 mM Tris-hydrochloride buffer, pH 8.2) of the anti-hCG monoclonal antibody obtained in (1-a) was spotted at the center of the membrane and settled for 30 minutes in the thermostat described above. To block portions other than the antibody-spotted areas, the membrane was immersed in 5% BSA solution followed by warming at 37°C for 45 minutes. The membrane was washed twice with Tris-hydrochloride buffer. After immersing in 0.1% BSA-containing Tris-hydrochloride buffer, the membrane was drained and dried to prepare white anti-hCG-specific monoclonal antibody-immobilized membrane.

(1-d) Preparation of anti-hCG antibody-sensitized latex reagent

The anti-hCG polyclonal antibody, 10 mg, obtained in (1-b) was dissolved in 4 ml of glycine buffer (pH 8.2). Then, 1.0 ml of red polystyrene latex (solid content of 10%, manufactured by Japan Synthetic Rubber Co., Ltd., particle diameter of 0.303 $\mu$m) was dropwise added to the solution with stirring. Stirring was continued for 30 minutes at room temperature and for 30 minutes at 56°C. After cooling, the reaction mixture was centrifuged. The obtained precipitates were suspended in 10 ml of glycine buffer. After repeating the centrifuging operation twice, the precipitates were suspended in 10 ml of 1% BSA-containing glycine buffer. After stirring at room temperature for an hour, centrifugation was carried out. After repeating the washing operation 3 times, the precipitates were suspended in 20 ml of 0.1% BSA-containing glycine buffer to prepare anti-hCG polyclonal antibody-sensitized latex.

(1-e) Assay method

The anti-hCG-specific monoclonal antibody-immobilized membrane prepared in (1-c) was placed on a porous resin and the resin was set in a holding frame, which was provided for reaction. Using 0.1% BSA-containing Tris-hydrochloride buffer, standard hCG solutions having various concentrations were prepared and 200 $\mu$l each of the solutions was dropped at the center of the membrane and absorbed therein. Then, two drops (about 70 $\mu$l) of the anti-hCG polyclonal antibody-sensitized latex reagent obtained in (1-d) were dropped thereon. Immediately after the reagent was absorbed into the membrane, 0.1% BSA-containing Tris buffer (pH 8.2) was dropped and the unreacted latex was washed off and removed below the membrane, whereby the presence or absence of colored spots was visually determined. For control, 0.1% BSA-containing Tris buffer was used instead of the hCG solution. The results are shown in Table 1.

EP 0 396 801 A1

Table 1

| Concentration of hCG (iu/ml) | 10 | 1 | 0.5 | 0.1 | 0.05 | 0.025 | 0.01 | Control |
|---|---|---|---|---|---|---|---|---|
| Judgment | + | + | + | + | + | ± | - | - |

In the table:

+ indicates that red spots were observed;

- indicates that no color was noted; and,

± indicates that a slight color was observed.

These symbols have also the same significance in the following examples.

Next, 10 urine samples collected from 10 pregnants were examined by comparing with the prior art method (latex agglutination method; sensitivity of 200 miu/ml). The hCG concentration in each urine from the pregnants was quantitatively determined by radioimmunoassay (RIA). The results are shown in Table 2.

Table 2

| Urine Sample No. | 1 | 2 | 3 | 4 | 5 | 6 | 7 | 8 | 9 | 10 |
|---|---|---|---|---|---|---|---|---|---|---|
| RIA value (miu/ml) | 1050 | 700 | 210 | 80 | 65 | 830 | 36 | 173 | 55 | 138 |
| Invention | + | + | + | + | + | + | + | + | + | - |
| Prior art | + | + | ± | - | - | + | - | - | - | - |

As is clear from the results, the method of the present invention has a higher sensitivity than in the latex agglutination method. The reaction operation can be performed within 2 minutes.

Example 2

(2-a) Preparation of anti-hLH-specific monoclonal antibody

Using hLH as the antigen, Balb/c mouse was immunized as in Example (1-a). Using the spleen cells, cell fusion was performed to obtain a fused cell line secreting hLH-specific monoclonal antibody and a fused cell line secreting a monoclonal antibody capable of recognizing α-subunit cross-reactive with hCG, hLH and hFSH. Each cell line was intraperitoneally administered to Balb/c mouse previously administered with puristan to cause ascites tumor. The ascites were collected and subjected to fractionation with ammonium sulfate and then Affigel-Protein A MAPS KIT to purify. By freeze drying, a white powdery monoclonal antibody was obtained. The resulting anti-hLH-specific monoclonal antibody was used for membrane immobilization and the monoclonal antibody capable of recognizing α-subunit was used to sensitize the latex particles.

(2-b) Preparation of anti-hLH monoclonal antibody-immobilized membrane

White nitrocellulose membrane (manufactured by Sartorius Inc.) having a pore diameter of 8 μm was cut into a circle having a diameter of 20 mm and the anti-hLH antibody obtained in (2-a) was immobilized in a manner similar to Example (1-b) to prepare the anti-hLH antibody-immobilized membrane.

(2-c) Preparation of anti-hLH monoclonal antibody-sensitized latex reagent

The monoclonal antibody, 2 mg, capable of recognizing α-subunit obtained in (2-a) was dissolved in 4 ml of glycine buffer (pH 8.2). Then, 1.0 ml of blue polystyrene latex (solid content of 10%, manufactured by

7

Japan Synthetic Rubber Co., Ltd., particle diameter of 0.653 μm) was dropwise added to the solution with stirring. After stirring for an hour at room temperature, the mixture was warmed at 56°C for 30 minutes. After cooling, the reaction mixture was treated in a manner similar to Example (1-d) to prepare anti-hLH monoclonal antibody-sensitized latex reagent.

(2-d) Assay method

The anti-hLH monoclonal antibody-immobilized membrane was placed on an adsorbent made of a porous resin and the resin was set in a holding frame in a manner similar to Example (1-e), which was provided for reaction. Using 0.1% BSA-containing Tris buffer, hLH solutions having various concentrations were prepared and 500 μl each of the solutions was dropped onto the membrane and absorbed therein. Then, three drops (about 100 μl) of the anti-hLH antibody-sensitized latex reagent obtained in (2-c) were dropped thereon. After the reagent was absorbed into the membrane, 0.1% BSA-containing Tris buffer was dropped and the unreacted latex was washed off and removed. For control, 0.1% BSA-containing Tris buffer was used instead of the hLH solution and treated in a similar manner. The results are shown in Table 3.

Table 3

| hLH Solution (iu/ml) | 1 | 0.5 | 0.25 | 0.1 | 0.05 | 0.025 | Control |
|---|---|---|---|---|---|---|---|
| Judgment | + | + | + | + | + | - | - |

As is clear from the results, the reagent having a measurement sensitivity as extremely high as 0.05 iu/ml (50 miu/ml) was obtained. The time required for the assay was within 2 minutes.

Example 3

(3-a) Preparation of anti-hFSH-specific monoclonal antibody

Using hFSH as the antigen, Balb/c mouse was immunized as in Example (1-a). Using the spleen cells, cell fusion was performed to obtain a fused cell line secreting anti-hFSH-specific monoclonal antibody. This cell line was intraperitoneally administered to Balb/c mouse previously treated with puristan to cause ascites tumor. The ascites were collected and purified by fractionation with ammonium sulfate and then with Affigel-Protein A MAPS KIT. By freeze drying, white powdery anti-hFSH-specific monoclonal antibody was obtained. This monoclonal antibody was used to sensitize the latex particles.

(3-b) Preparation of anti-HMG antibody

HMG, 1 mg, was subcutaneously administered to rabbit at the back together with complete Freund's adjuvant and further administered twice in 3 weeks interval subcutaneously and between the paws. Whole blood was collected 3 weeks after the final immunization and sera were separated therefrom to give the antisera. After the obtained antisera were immobilized at 56°C for 30 minutes, IgG was purified by fractionation with ammonium sulfate, DEAE-cellulose chromatography and gel filtration by Sephadex G200. Freeze drying gave anti-HMG antibody. The obtained anti-HMG antibody was used for immobilization of membrane.

(3-c) Preparation of anti-HMG antibody-immobilized membrane

Black membrane (manufactured by Toyo Filter Paper Co., Ltd.) having a pore diameter of 3.0 μm was cut into a circle having a diameter of 20 mm and the anti-HMG antibody obtained in Example (3-b) was immobilized in a manner similar to Example (1-c) to prepare the anti-HMG antibody-immobilized membrane.

(3-d) Preparation of anti-hFSH-specific monoclonal antibody-sensitized latex reagent

The anti-hFSH-specific monoclonal antibody, 1 mg, obtained in (3-a) was dissolved in 4 ml of glycine buffer (pH 8.2). Then, 1.0 ml of white polystyrene latex (solid content of 10%, manufactured by Dow Chemical Co., Ltd., particle diameter of 0.103 μm) was dropwise added to the solution with stirring. After stirring for an hour at room temperature, the mixture was warmed at 56°C for 30 minutes. After cooling, the reaction mixture was washed in a manner similar to Example (1-d) and suspended in 20 ml of 0.1% BSA-containing glycine buffer (pH 8.2) to prepare anti-hFSH-specific monoclonal antibody- sensitized latex reagent.

(3-e) Assay method

Using the anti-HMG antibody-immobilized membrane obtained in (3-c) and the anti-hFSH-specification monoclonal antibody-sensitized latex reagent, hFSH was measured in a manner similar to Example (1-e). The results are shown in Table 4.

Table 4

| hFSH Solution (iu/ml) | 1 | 0.5 | 0.25 | 0.1 | 0.05 | 0.025 | 0.01 | Control |
|---|---|---|---|---|---|---|---|---|
| Judgment | + | + | + | + | + | + | - | - |

Example 4

(4-a) Preparation of anti-hPL antibody

hPL, 1 mg, was subcutaneously administered to rabbit at the back together with complete Freund's adjuvant and one month after, further adminstered twice in 3 weeks interval subcutaneously and between the paws. Whole blood was collected 3 weeks after the final immunization and sera were separated therefrom to give the antisera. After the obtained antisera were immobilized at 56°C for 30 minutes, IgG was purified by fractionation with ammonium sulfate, DEAE-cellulose chromatography and gel filtration by Sephadex G200. Freeze drying gave white anti-hPL antibody powders.

(4-b) Preparation of anti-hPL antibody-immobilized membrane

A dry membrane made of Paul immunodyne immunoaffinity membrane (manufactured by Paul Biomedical Product Corporation, white) having a pore diameter of 3 μm was cut into a circle having a diameter of 20 mm and 5 μl each of a phosphate buffer solution (pH 7.2) containing 10 mg/ml of the anti-hPL antibody obtained in (4-a) was spotted onto the membrane at the center thereof followed by drying at room temperature. After washing 3 times with phosphate buffer (pH 7.2), the membrane was immersed in 5% BSA-containing phosphate buffer (pH 7.2) followed by warming at 37°C for 45 minutes. Then, the membrane was washed 3 times with the buffer described above and then dried at room temperature to give anti-hPL antibody-immobilized membrane.

(4-c) Preparation of anti-hPL antibody-sensitized latex reagent

The anti-hPL antibody, 5 mg, obtained in (4-a) was dissolved in 4 ml of glycine buffer (pH 8.2). Then, 1.0 ml of black polystyrene latex (solid content of 10%, manufactured by Japan Synthetic Rubber Co., Ltd., particle diameter of 0.209 μm) was dropwise added to the solution with stirring. After stirring for an hour at room temperature, the mixture was warmed at 56° C for 30 minutes. After cooling, the reaction mixture was washed in a manner similar to Example (1-d) and suspended in 20 ml of 0.1% BSA-containing glycine buffer to prepare anti-hPL antibody-sensitized latex reagent.

(4-d) Assay method

Using the anti-hPL antibody-immobilized membrane obtained in (4-b) and the anti-hPL antibody-sensitized latex reagent obtained in (4-c), hPL was measured in a manner similar to Example (1-e). The results are shown in Table 5.

Table 5

| Concentration of hPL (μg/ml) | 1 | 0.5 | 0.2 | 0.1 | 0.05 | 0.02 | 0.005 | Control |
|---|---|---|---|---|---|---|---|---|
| Judgment | + | + | + | + | + | + | - | - |

While the invention has been described in detail and with reference to specific embodiments thereof, it is apparent to one skilled in the art that various changes and modifications can be made therein without departing from the spirit and the scope of the invention.

**Claims**

1. An immunological assay for an antigen in a liquid sample which comprises:
reacting an antigen contained in a liquid sample with a first antibody which is carried on a porous carrier membrane having a pore diameter larger than a particle diameter of latex particles; and,
reacting the formed antigen-antibody complex with a second antibody which is carried on latex particles having a color different from that of said porous carrier membrane and has an antigenic determinant different from said first antibody.

2. An immunological assay according to claim 1, wherein said first antibody is a monoclonal antibody and said second antibody is a monoclonal antibody having an antigenic determinant different from said first monoclonal antibody.

3. An immunological assay according to claim 1, wherein said first antibody is a monoclonal antibody and said second antibody is a polyclonal antibody to the same antigen.

4. An immunological assay according to claim 1, wherein said first antibody is a polyclonal antibody and said second antibody is a monoclonal antibody.

5. An immunological assay according to claim 1, wherein said first antibody is a polyclonal antibody and said second antibody is a polyclonal antibody.

6. An immunological assay according to claim 1, wherein said porous carrier membrane has a pore diameter of about 0.2 to about 10 μm.

7. An immunological assay according to claim 6, wherein said porous carrier membrane has a pore diameter of about 0.45 to about 10 μm.

8. An immunological assay according to claim 1, wherein said latex particles have a particle diameter of about 0.01 to about 2 μm.

9. An immunological assay according to claim 8, wherein said latex particles have a particle diameter of about 0.05 to about 0.8 μm.

10. An immunological assay according to claim 1, wherein a ratio of a pore diameter of said porous carrier membrane to a diameter of said latex particles is in a range of 3 : 1 to 15 : 1.

11. An immunological assay according to claim 1, wherein said porous carrier membrane is colored to a color selected from the group of white, yellow and blue, and said latex particles are colored to a color other than said color of the porous carrier membrane.

12. A kit for an immunological assay for an antigen in a liquid sample comprising:

a first antibody-carrying membrane in which a first antibody is carried on a porous carrier membrane having a pore diameter larger than a particle diameter of latex particles; and,

a second antibody-carrying latex particles in which a second antibody is carried on latex particles having a color different from that of said porous carrier membrane and has an antigenic determinant different from said first antibody.

13. A kit for an immunological assay according to claim 12, wherein said first antibody-carrying membrane is anti-human chorionic gonadotropin (hCG) monoclonal antibody-carrying nitrocellulose membrane and said second antibody-carrying latex particles are anti-hCG polyclonal antibody-carrying latex particles.

14. A kit for an immunological assay according to claim 12, wherein said first antibody-carrying membrane is anti-prostate acidic phosphatase (PAP) polyclonal antibody-carrying membrane filter paper and said second antibody-carrying latex particles are anti-PAP monoclonal antibody-carrying latex particles.

15. A kit for an immunological assay according to claim 12, wherein said first antibody-carrying membrane is anti-α-fetroprotein (AFP) monoclonal antibody-carrying filter paper and said second antibody-carrying latex particles are anti-AFP monoclonal antibody-carrying latex particles.

16. A kit for an immunological assay according to claim 12, wherein said first antibody-carrying membrane is anti-carcinoembryonic antigen (CEA) polyclonal antibody-carrying immunodyne immunoaffinity membrane and said second antibody-carrying latex particles are anti-CEA monoclonal antibody-carrying latex particles.

17. A kit for an immunological assay according to claim 12, wherein said first antibody-carrying membrane is anti-fibrin/fibrinogen degradation product (FDP) monoclonal antibody-carrying nitrocellulose membrane and said second antibody-carrying latex particles are anti-FDP monoclonal antibody-carrying latex particles.

18. A kit for an immunological assay according to claim 12, wherein said first antibody-carrying membrane is anti-human growth hormone (hGH) polyclonal antibody-carrying filter paper and said second antibody-carrying latex particles are anti-hGH monoclonal antibody-carrying latex particles.

19. A kit for an immunological assay according to claim 12, wherein said first antibody-carrying membrane is anti-human lutenizing hormone (hLH) monoclonal antibody-immobilized membrane and said second antibody-carrying latex particles are anti-hLH monoclonal antibody-carrying latex particles.

20. A kit for an immunological assay according to claim 12, wherein said first antibody-carrying membrane is anti-human menopausal gonadotropin (HMG) antibody-immobilized membrane and said second antibody-carrying latex particles are anti-hFSH-specific monoclonal antibody-carrying latex particles.

21. A kit for an immunological assay according to claim 12, wherein said first antibody-carrying membrane is anti-human placental lactogen (hPL) antibody-immobilized membrane and said second antibody-carrying latex particles are anti-hpL antibody-carrying latex particles.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 310 862 (BECTON DICKINSON & CO.) <br> * Page 4, line 11 - page 5, line 38; page 7, lines 18-48; page 8, lines 1-2; page 9, lines 1-15; page 10, lines 36-55 * | 1,3,5-10,12 | G 01 N 33/538 <br> G 01 N 33/58 <br> G 01 N 33/546 <br> G 01 N 33/74 <br> G 01 N 33/68 |
| X | EP-A-0 302 673 (BECTON DICKINSON & CO.) <br> * Column 1, lines 21-44; column 4, lines 10-18; column 5, lines 31-45; column 11, lines 1-52 * | 1,3,6,7,12 | |
| A | | 13,19 | |
| X | EP-A-0 291 176 (BECTON DICKINSON & CO.) <br> * Column 1, line 48 - column 2, line 29; column 7, lines 19-27 * | 1,12 | |
| X | WO-A-8 505 451 (HYBRITECH INC.) <br> * Page 3, line 5 - page 4, line 28; page 5, lines 16-32; page 8, lines 5-23; page 10, line 33 - page 11, line 6 * | 1,2,12 | |
| Y | | 3-10,13-16,19 | TECHNICAL FIELDS SEARCHED (Int. Cl.5) <br><br> G 01 N |
| Y | EP-A-0 239 318 (FISHER SCIENTIFIC CO.) <br> * page 3, line 5 - page 9, line 26 * | 3-10,13-16,19 | |
| Y | EP-A-0 310 406 (E-Y lABORATORIES INC.) <br> * Column 2, line 59 - column 3, line 11; column 9, line 19 - column 10, line 19; column 13; column 15, lines 36-47 * <br> -/- | 1-3,8-10,12,13,19,21 | |

The present search report has been drawn up for all claims

| Place of search | Date of completion of the search | Examiner |
|---|---|---|
| THE HAGUE | 03-01-1990 | HITCHEN C.E. |

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 293 779 (DAIICHI PURE CHEMICALS CO., LTD) <br> * Page 3, line 46 - page 4, line 50 * | 1-3,8-10,12,13,19,21 | |
| A | EP-A-0 312 394 (QUADRA LOGIC TECHNOLOGIES INC.) <br> * Column 3, line 9 - column 4, line 31 * | 1 | |

TECHNICAL FIELDS SEARCHED (Int. Cl.5)

The present search report has been drawn up for all claims

| Place of search | Date of completion of the search | Examiner |
|---|---|---|
| THE HAGUE | 03-01-1990 | HITCHEN C.E. |